# EUROPEAN PATENT APPLICATION

(11) **EP 4 574 199 A1**
(43) Date of publication of application: **25.06.2025**
(21) Application number: 23854454.8
(22) Date of filing: 15.08.2023
(51) Int. Cl.: A61M 60/216, A61M 60/157, A61M 60/414, A61M 60/135, A61M 60/237, A61M 60/205, A61M 25/08

(54) **CATHETER PUMP**

(30) Priority: 15.08.2022 CN 202210976332; 09.12.2022 CN 202211578294
(71) Applicant: Magassist Co., Ltd., Suzhou, Jiangsu 215163 (CN)
(72) Inventor: ZHANG, Jialiang, Suzhou, Jiangsu 215163 (CN); LOGAN, Thomas George, Suzhou, Jiangsu 215163 (CN); HSU, Chiahao, Suzhou, Jiangsu 215163 (CN)
(74) Representative: Winter, Brandl - Partnerschaft mbB
(86) International application number: PCT/CN2023/113206
(87) International publication number: WO 2024/037554

(57) **Abstract**

A catheter pump (1000), a driving catheter handle (204) thereof including a coupling housing (2045) detachably connected to a motor (102). The proximal end of a driving shaft (202) is connected to a rotor (2046), the rotor (2046) transmits rotational power of the motor (102) to the driving shaft (202), thus driving an impeller (2052) of a pump head (205) to rotate. The coupling housing (2045) is internally provided with an accommodating chamber (2049) supporting the rotor (2046) therein and communicating with the catheter (201), and is provided with a flushing liquid inlet (2043) and a flushing liquid outlet (246) communicating with the accommodating chamber (2049) are provided. The flushing liquid inlet (2043) and the flushing liquid outlet (246) are in communication with a flushing liquid inlet tube (501) and a flushing liquid outlet tube (502), respectively, the flushing liquid outlet tube (502) is in communication with the flushing liquid inlet tube (501), the flushing liquid outlet tube (502), the flushing liquid inlet tube (501), and the accommodating chamber (2049) form a cooling circulation loop, a circulation driving member (503) is provided on the cooling circulation loop, and the cooling circulation loop is connected to a pressure maintenance module (600) for maintaining pressure of flushing liquid in the cooling circulation loop.

## Description

### Technical Field

The present invention relates to a catheter pump.

### Background

Heart disease is a health issue with a high mortality rate. Mechanical circulatory support systems are increasingly configured by doctors to treat heart failure. Treatment for acute heart failure requires a device capable of quickly providing support for a patient, and doctors wish to quickly and minimally invasively deploy a treatment regimen.

Mechanical circulatory support (MCS) systems and ventricular assist devices (VADs) gain increased acceptance in the treatment of acute heart failure. For example, in the treatment of acute myocardial infarction (MI) or compensated heart failure, MCSs and VADs are configured to stabilize patients after cardiogenic shock, or MCSs and VADs are configured during high-risk percutaneous coronary intervention (PCI) to provide support for patients. One example of MCS systems is a rotary catheter pump placed percutaneously via a catheter.

In conventional approaches, a catheter pump is inserted into the body and is connected to the cardiovascular system (e.g. being connected to the left ventricle and the ascending aorta) to assist in the pump function of the heart. Other known applications include pumping venous blood from the right ventricle to the pulmonary artery to support the right side of the heart. In general, acute circulatory support devices are configured to reduce the load of the myocardium over a period of time, to stabilize patients prior to heart transplantation or provide sustained support.

As in the known embodiments of the application with the publication no. CN 113856036 A, a catheter pump that achieves a small interventional size, that is, using an external motor, is provided. The general working principle of the catheter pump is as follows: an external motor transmits rotational power to a distal impeller by means of a driving shaft passing through a catheter, the distal impeller rotates to provide flow power for blood, and the blood is pumped from the left ventricle to the aorta. During power transmission by the impeller, since there are a plurality of rotating components, such as the driving shaft and proximal and distal bearings that support the impeller, during operation of the catheter pump, it needs to inject flushing liquid into the catheter, so as to lubricate and cool the plurality of rotating components.

To avoid leakage of flushing liquid, particularly to prevent flushing liquid from entering a motor, at present catheter pumps employ non-contact power transmission mechanisms, including a magnetic coupling solution as provided in the application with the publication no. CN 101820933 B and an eddy-current coupling solution as provided in the application with the publication no. CN 114452527 A and in the application with the publication no. CN 216061675 U. A wall for liquid isolation is provided between a driving member and a driven member (in a magnetic coupling solution, both being magnets, and in an eddy-current coupling solution, the two being a magnet and a conductor, respectively), sealing flushing liquid, such that the flushing liquid may only flow towards a distal end and flush a driving shaft and proximal and distal bearings, and does not enter a motor.

The driving member is driven by the motor to rotate, the driven member is provided on a rotor shaft and is supported by the rotor shaft, and the rotor shaft is fixedly connected to the proximal end of the driving shaft circumferentially. In this way, by means of the effect of magnetic coupling between the driving member and the driven member, the rotation of the motor is transmitted to the rotor shaft and further to the driving shaft and an impeller.

A rotor comprising the driven member and the rotor shaft is provided in a driving catheter handle detachably connected to the motor, and the rotor further comprises a protective layer covering the driven member and an end cover axially defining the driven member. The rotor shaft is rotatably supported in the driving catheter handle by means of two bearings. A flushing liquid interface is provided on the driving catheter handle. In this way, flushing liquid first enters the driving catheter handle and then enters a catheter. Thus, the rotor is submerged in the flushing liquid. Thus, the flushing liquid also serves to lubricate and cool the two bearings which support the rotor shaft.

It's worth noting that the torque of a non-contact power transmission mechanism is inversely correlated with the distance between a driving member and a driven member. This is particularly clear in an eddy-current coupling solution. Thus, in order to increase the torque of power transmission, it is feasible to reduce the distance between a driving member and a driven member by increasing the diameter of a rotor.

However, increasing the diameter of a rotor would simultaneously reduce the distance between the outer wall of the rotor and the inner wall of a driving catheter handle. In practice, it is found that, since a rotor is submerged in flushing liquid, when the rotor rotates at a high speed, the flushing liquid would be driven by the rotor in the narrow space in a driving catheter handle due to viscosity of the liquid, generating a huge eddy-current loss, further causing great temperature rise of the flushing liquid. Since flushing liquid would at least partially enter the human body via a catheter, great temperature rise of the flushing liquid adversely affects the human body.

Thus, how to solve temperature rise of flushing liquid is a technical problem to be solved urgently.

### Summary

To this end, some embodiments of the present invention provide a catheter pump, for at least partially solving the described problem.

In an embodiment of the present invention, a catheter pump is provided:
The catheter pump includes a driving assembly, a working assembly, a cooling circulation module, and a pressure maintenance module.

The driving assembly includes a motor.

The working assembly includes a catheter, a driving shaft passing through the catheter, a driven member connected to the proximal end of the driving shaft, and a driving catheter handle and a pump head connected to the proximal end and the distal end of the catheter, respectively. The pump head includes a pump housing connected to the distal end of the catheter, and an impeller housed in the pump housing. The impeller is connected to the distal end of the driving shaft so as to be driven to rotate to pump blood. The driving catheter handle includes a coupling housing connected to the proximal end of the catheter and detachably connected to the motor, and a rotor which is able to be driven by the motor, and the proximal end of the driving shaft is connected to the rotor. An accommodating chamber for rotatably supporting the rotor therein is formed in the coupling housing, the coupling housing is provided with a flushing liquid inlet and a flushing liquid outlet which are in communication with the accommodating chamber, and the accommodating chamber is in communication with the catheter. The flushing liquid inlet is in communication with a flushing liquid source, and flushing liquid entering the accommodating chamber via the flushing liquid inlet is divided into two parts: a first part enters the catheter, and a second part passes through the rotor via the flushing liquid and then is discharged from the flushing liquid outlet.

The first part of the flushing liquid is finally entirely discharged via the pump head to the human body. This means that the first part of the flushing liquid entering via the catheter does not back flow, but enters the human body entirely. The so-called backing flow refers to the flushing liquid flowing back from the distal end (substantially at the position of the pump head) to the proximal end (in an embodiment, at the position of the driving catheter handle) again via the catheter or the driving shaft.

The first part of the flushing liquid is mainly discharged at the distal end (in an embodiment, at the position of the pump head) via two parts, i.e. the distal end of the catheter and the distal end of the driving shaft.

The catheter pump includes a first flow path for the flow of the first part of the flushing liquid, and the first flow path is defined by the inner spaces of structures such as the flushing liquid inlet, the accommodating chamber, the catheter, and the pump head. Thus, the flow path of the first part of the flushing liquid enters the accommodating chamber via the flushing liquid inlet, then enters the catheter from the accommodating chamber, and finally flows out via the pump head.

The catheter pump also includes a second flow path for the flow of the second part of the flushing liquid, and the second flow path is defined by the inner spaces of structures such as the flushing liquid inlet, the accommodating chamber, and the flushing liquid outlet. Thus, the flow path of the second part of the flushing liquid enters the accommodating chamber via the flushing liquid inlet, and then flows out via the flushing liquid outlet.

It's worth noting that there is no obvious interface between the first part of the flushing liquid and the second part of the flushing liquid during flowing. The description adopted in the present invention is merely for the sake of brevity. For convenience, the flushing liquid finally entering the catheter is defined as the first part of the flushing liquid and the flushing liquid finally discharged via the flushing liquid outlet is defined as the second part of the flushing liquid.

In some embodiments of the present invention, the flushing liquid is diverted in the driving catheter handle. The first part of the flushing liquid entering the catheter is able to lubricate and cool rotating components such as the driving shaft and bearings when flowing towards the position of the distal pump head. The second part of the flushing liquid flowing out via the flushing liquid outlet is able to carry away high temperature of the rotor caused by high speed, avoiding high temperature of the flushing liquid.

The cooling circulation module includes a flushing liquid inlet tube in communication with the flushing liquid inlet and a flushing liquid outlet tube in communication with the flushing liquid outlet, the flushing liquid outlet tube is in communication with the flushing liquid inlet tube, and the flushing liquid outlet tube, the flushing liquid inlet tube, and the accommodating chamber form a cooling circulation loop. The cooling circulation module further includes a circulation driving member provided on the cooling circulation loop to drive the flushing liquid to flow.

The pressure maintenance module is connected to the cooling circulation loop, and is configured for maintaining the pressure of the flushing liquid in the cooling circulation loop to be greater than the ambient pressure of the pump head during operation.

In the cooling circulation loop formed by means of the flushing liquid inlet tube, the flushing liquid outlet tube, and the accommodating chamber, most amount of flushing liquid circulates in the cooling circulation loop by means of the circulation driving member, preventing the flushing liquid from forming a flow dead area in the accommodating chamber, further avoiding high temperature caused by eddy-current loss of the flushing liquid when the rotor rotates at a high speed in the accommodating chamber. Moreover, the pressure maintenance module maintains the pressure of the flushing liquid in the cooling circulation loop to be greater than the ambient pressure of the pump head, such that the flushing liquid is able to enter the human body via the catheter. Since the problem of high temperature of the flushing liquid is solved, the flushing liquid entering the human body does not cause discomfort.

In an embodiment, the circulation driving member is a first pump, which is able to be arranged on the flushing liquid inlet tube or the flushing liquid outlet tube; in another embodiment, the flushing liquid inlet tube and the flushing liquid outlet tube arrange a first pump respectively.

As described above, a part of the flushing liquid enters the catheter and the other part of the flushing liquid flows out via the flushing liquid outlet during flowing. Moreover, the amount of flushing liquid entering the catheter (the first part of the flushing liquid) is less than the amount of flushing liquid flowing out via the flushing liquid outlet (the second part of the flushing liquid). The term "amount" may refer to a volume or a flow rate. A flow rate is described herein.

The described distribution of the liquid flow rate is able to be realized by controlling the pressure maintenance module to maintain the pressure of the flushing liquid in the cooling circulation loop to be greater than the ambient pressure of the pump head during operation.

The pressure maintenance module includes the flushing liquid source in communication with the cooling circulation loop by means of a replenishment line, and a second pump provided on the replenishment line to replenish, into the cooling circulation loop, flushing liquid provided by the flushing liquid source. In this way, the power required for flushing liquid circulation and the pressure required for flushing liquid pressure maintenance are implemented by the circulation driving member and the second pump, respectively.

Since technology of pump control is comparatively mature, in cases where the first pump is configured as the circulation driving member, the power required for the flushing liquid circulation and the pressure required for flushing liquid maintenance are provided by the first pump and the second pump, respectively, such that accurate control of the flow rate of the flushing liquid entering the human body is able to be achieved.

In order to facilitate line connection, the cooling circulation module further includes a temporary storage container, and the flushing liquid inlet tube, the flushing liquid outlet tube, and the replenishment line all communicate with the temporary storage container by means of a Luer taper. Furthermore, in order to ameliorate to a certain degree unstable (decreasing) pressure of the flushing liquid caused by tube diameter expansion which may occur in a situation where the flushing liquid inlet tube and the flushing liquid outlet tube are flexible tubes, which finally causes an unstable flow rate of the flushing liquid entering the human body, the temporary storage container is able to withstand at least pressure compensation by the pressure maintenance module without a change in volume, and preferably is a solid tank or a three-way connector having no change in volume.

In order to prevent gas precipitating from the flushing liquid due to high-speed rotation of the rotor from entering the human body, a bubble filter is provided on the cooling circulation loop for catching or filtering precipitated bubbles. The bubble filter is preferably arranged on the flushing liquid inlet tube, and is able to substantially prevent bubbles from entering the human body via the catheter.

### Brief Description of the Drawings

Fig. 1 is a schematic diagram of the three-dimensional structure of a catheter pump according to embodiments of the present invention;
Fig. 2 is a sectional view of a pump head of the front-end portion of a working assembly of Fig. 1;
Fig. 3 is a sectional view of a driving assembly and a driving catheter handle in a separated state; and
Fig. 4 is a structural schematic diagram of a catheter pump with a pump as a circulation driving member and a flushing liquid high temperature solution corresponding to the third embodiment of the present invention.

### Detailed Description of the Embodiments

As used herein, the terms "proximal" and "rear", and "distal" and "front" are mentioned relative to a clinician operating a catheter pump. The terms "proximal" and "rear" refer to portions that are comparatively closer to a clinician, and the terms "distal" and "front" refer to portions that are comparatively further from the clinician. For example, a driving assembly is at the proximal end and the rear end and a working assembly is at the distal end and the front end. As another example, the proximal end of a component/assembly refers to the end comparatively proximal to the driving assembly and the distal end refers to the end comparatively proximal to the working assembly.

It should be understood that the terms "proximal", "distal", "rear", and "front" are defined for convenience of description. However, the catheter pump may be used in many directions and locations, thus the terms expressing relative positional relationships are not limited or absolute.

The catheter pump in the embodiments of the present invention is able to at least partially assist the blood pumping function of the heart, and achieve the effect of at least partially reducing the burden of the heart. In one illustrative scenario, the catheter pump may be used for assisting the left ventricle, the working portion thereof (specifically, the pump head hereinafter) may be intervened into the left ventricle, and the pump head, when in operation, may pump blood from the left ventricle into the ascending aorta.

It is worth noting that the example hereinabove in which the catheter pump is used for assisting the left ventricular is merely one possible application scenario for the present catheter pump. In other possible and not expressly excluded scenarios, the catheter pump may also be used for assisting the right ventricle, the pump head may be intervened into the right ventricle, and the pump head, when in operation, pumps blood from the vein into the right ventricle.

The following is set forth primarily with the use of the present catheter pump for assisting the left ventricle. However, on the basis of the description hereinabove, it can be determined that the scope of protection of embodiments of the present invention is not limited thereto.

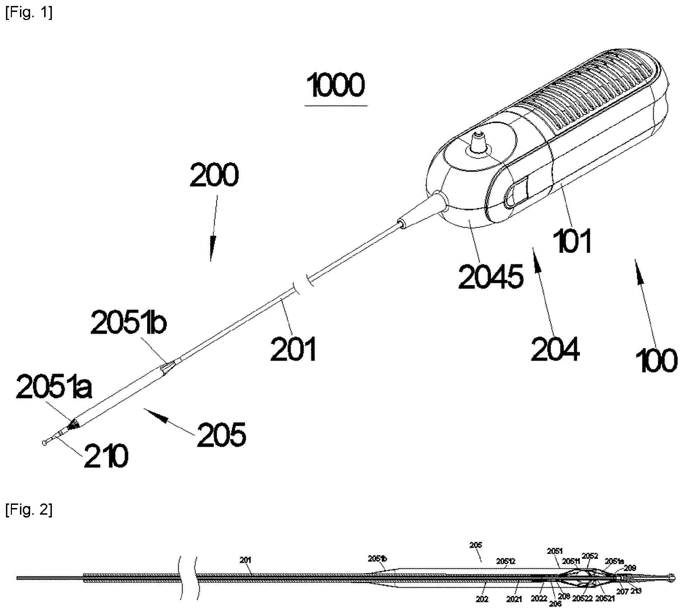

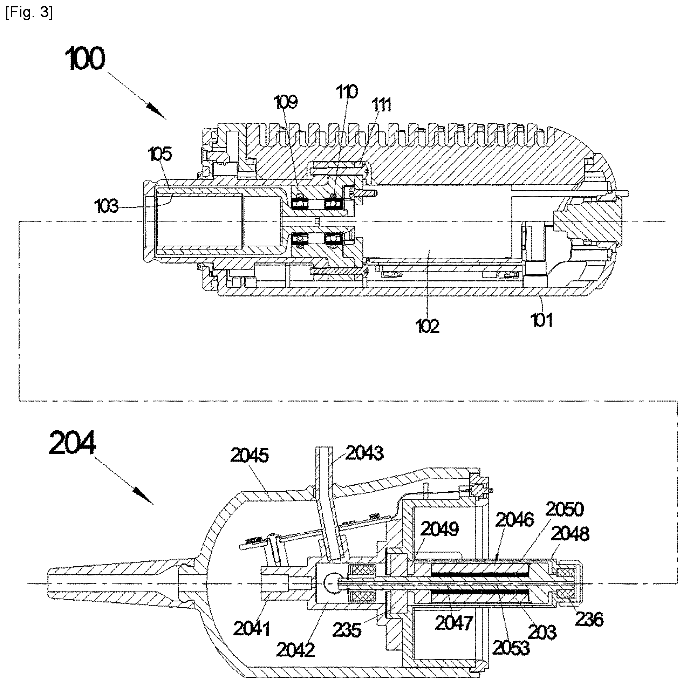

As shown in Fig. 1, a catheter pump 1000 includes a driving assembly 100 and a working assembly 200. In view of Fig. 3, the driving assembly 100 includes a motor housing 101, a motor 102 housed in the motor housing 101, and a driving member 103 driven by the motor 102. In view of Fig. 2, the working assembly 200 includes a catheter 201, a driving shaft 202 passing through the catheter 201, a driven member 203 connected to the proximal end of the driving shaft 202, and a driving catheter handle 204 and a pump head 205 connected to the proximal end and the distal end of the catheter 201, respectively. The pump head 205 includes a pump housing 2051 provided with an inlet 2051a and an outlet 2051b, and an impeller 2052 housed in the pump housing 2051, the impeller being connected to the distal end of the driving shaft 202. When rotating, the impeller 2052 suctions blood into the pump housing 2051 via the inlet 2051a, and then pumps the blood out of the pump housing 2051 via the outlet 2051b.

In one embodiment, the pump housing 2051 includes a stent 20511 and an elastic coating 20512 covering the stent 20511. The metal grid structure of the stent 20511 has mesh design, the coating 20512 covers the middle part and the rear end part of the stent 20511, and the meshes of the part of the front end of the stent 20511 not covered by the coating 20512 form the inlet 2051a. The rear end of the coating 20512 covers the outside of the distal end of the catheter, and the outlet 2051b is an opening formed at the rear end of the coating 20512. The impeller 2052 includes a hub 20521 and blades 20522 supported on the outer wall of the hub 20521. The blades 2052 are made of a flexible material, and form the collapsible pump head 205 together with the stent 20511 made of a nickel and titanium memory alloy and the coating 20512.

In other embodiments, the pump head 205 is non-collapsible. Accordingly, the pump housing 2051 is a metal casing which is not be able to radially collapsed or self-expand. The impellers 2052 are also made of a rigid but biocompatible material.

The driving shaft 202 includes a bendable flexible shaft 2021 and a rigid shaft 2022 connected to the distal end of the flexible shaft 2021, the flexible shaft 2021 passing through the catheter 201, the rigid shaft 2022 passing through a hollow channel of the hub 20521, and the outer wall of the rigid shaft 2022 being fixed to the inner wall of the hollow channel of the hub 20521 by means of adhesion.

The proximal end of the stent 20511 is connected to a proximal bearing chamber 206, and the distal end of the stent 20511 is connected to a distal bearing chamber 207, and the proximal bearing chamber 206 is provided with a proximal bearing 208, and the distal bearing chamber 207 is provided with a distal bearing 209. The proximal end of the rigid shaft 2022 pass through the proximal bearing 208, and the distal end of the rigid shaft 2022 pass through the distal bearing 209. In this way, two ends of the rigid shaft 2022 are supported by the bearings 208 and 209, and the rigid shaft 2022 has high rigidity, such that the impeller 2052 is well retained in the pump housing 2051.

As shown in Fig. 3, the driving catheter handle 204 includes a coupling housing 2045, a flushing support 2041 is provided in the coupling housing 2045, and the flushing support 2041 defines a flushing chamber 2042. The proximal end of catheter 201 passes through the coupling housing 2045 to be connected to the flushing support 2041 and be in communication with the flushing chamber 2042. The driving catheter handle 204 is provided with a flushing liquid inlet 2043 passing through a side wall of the coupling housing 2045, and the inner end of the flushing liquid inlet 2043 is in communication with the flushing chamber 2042.

As described above, the magnetic coupling between the driving member 103 and the driven member 203 implements non-contact power transmission, and a liquid isolating wall 2048 is able to be provided between the driving member 103 and the driven member 203 or outside the driving member 203 to seal the flushing liquid, preventing the flushing liquid from entering the motor 102. In addition, the liquid isolating wall 2048 defines the flow direction of the flushing liquid, such that the flushing liquid only flows towards the distal end, that is, towards the working assembly 200, thereby lubricating and cooling the rotating components, such as the driving shaft 202 and bearings in the working assembly 200.

As shown in Fig. 3, the driving catheter handle 204 also includes a rotor 2046 rotatably provided inside the coupling housing 2045. The rotor 2046 includes a rotor shaft 2047 on which the driven member 203 is provided, and the proximal end of the driving shaft 202 is connected to the rotor shaft 2047. The liquid isolating wall 2048 is arranged in the coupling housing 2045 and is located at the rear end of the flushing support 2041, and the liquid isolating wall 2048 and the flushing support 2041 abut against each other and together define an accommodating chamber 2049 for housing the rotor 2046 therein.

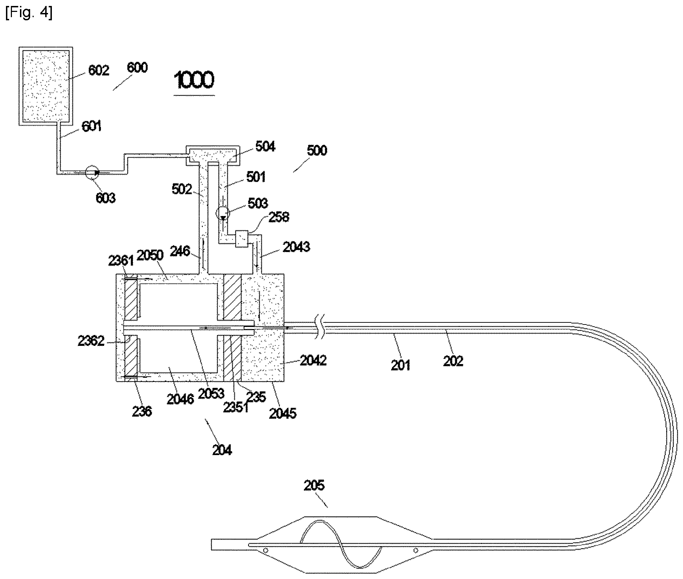

The accommodating chamber 2049 includes the flushing chamber 2042 defined by the flushing support 2041, and a liquid isolating chamber 2050 defined by the liquid isolating chamber 2048. The flushing chamber 2042 is in communication with the liquid isolating chamber 2050. In view of Fig. 4, the flushing chamber 2042 is in communication with a flushing liquid source 602 (flushing liquid saline solution, glucose solution, an anticoagulant, or any combination thereof) via the flushing liquid inlet 2043, the flushing liquid enters the flushing chamber 2042 via the flushing liquid inlet 2043, fills the accommodating chamber 2049, enters via the proximal end of the catheter 201 in communication with the flushing support 2041, and then flows towards the distal end via the catheter 201. In this process, the driving shaft 202 is lubricated. Moreover, the flexible shaft 2021 has a knitted structure, and liquid is able to penetrate into the flexible shaft. In this way, the flushing liquid flowing out of the flexible shaft 2021 flushes the proximal bearing 208 and flows out via a gap between the proximal bearing 208 and the rigid shaft 2022, achieving lubrication and cooling of the proximal bearing 208. The flushing liquid flowing forward inside the flexible shaft 2021 enters the rigid shaft 2022, and flows out via the distal end of the rigid shaft 2022. By being blocked by a sealing member 213 provided in the distal bearing chamber 207 and located on the distal side of the distal end of the rigid shaft 2022, the flushing liquid flows out reversely, i.e. flows out via a gap between the rigid shaft 2022 and the distal bearing 209, achieving lubrication and cooling of the distal bearing 209.

As shown in Fig. 4, in order to solve the problem of high temperature of the flushing liquid caused by high-speed rotation of the rotor 2046, the coupling housing 2045 is further provided with a flushing liquid outlet 246 in communication with the accommodating chamber 2049. The flushing liquid outlet 246 specifically is in communication with the liquid isolating wall 2048. The catheter pump 100 includes a cooling circulation module 500 that allows most amount of flushing liquid to circulate outside the body and a pressure maintenance module 600 that maintains the pressure of the flushing liquid in a cooling circulation loop.

The cooling circulation module 500 includes a flushing liquid inlet tube 501 in communication with the flushing liquid inlet 2043, and a flushing liquid outlet tube 502 in communication with the flushing liquid outlet 246. The flushing liquid outlet tube 502 and the flushing liquid inlet tube 501 are in communication with each other, and form with the accommodating chamber 2049 a cooling circulation loop, and the cooling circulation loop is provided with a circulation driving member 503 for driving the flushing liquid to flow.

The pressure maintenance module 600 is connected to the cooling circulation loop, and maintains the pressure of the flushing liquid in the cooling circulation loop to be greater than the ambient pressure of the pump head 205 during operation. In this way, the flushing liquid in the cooling circulation loop is able to partially enter the human body via the catheter 201, achieving cooling and lubrication of rotating components in the working assembly 200. Moreover, by being driven by the circulation driving member 503, the flushing liquid continuously circulates in the cooling circulation loop, carrying away high temperature of the flushing liquid caused by high-speed rotation of the rotor 2046, achieving cooling of the flushing liquid.

The front end of the pump head 205 (blood inlet 2051a) is intervened into the left ventricle during operation of the catheter pump 1000, and the rear end (blood outlet 2051b) is located in the aorta. Furthermore, when the impeller 2052 rotates to pump blood, the distal end of the catheter 201 is in a positive blood pressure region; thus, the ambient pressure of the pump head 205 during operation includes ventricular pressure, aortic pressure, positive blood pressure, etc. The resistance encountered by the flushing liquid entering the catheter 201 is greater than the resistance encountered by the flushing liquid flowing out via the flushing liquid outlet 246. This means that the flushing liquid needs to overcome the ambient pressure so as to enter the human body via the catheter 201.

In an embodiment, a first part of the flushing liquid, when flowing forward via the catheter 201 to the proximal bearing 208, needs to continue to flow forward after passing through bearing gaps. The bearing gaps specifically are a gap between the outer wall of the rigid shaft 2022 and the inner wall of the proximal bearing 208, and a gap between the outer wall of the rigid shaft 2022 and the inner wall of the distal bearing 209. During flowing forward, the first part of the flushing liquid encounters flow resistance at the bearing gaps, and the flow resistance is inversely correlated with the size of the bearing gaps. That is, the greater the bearing gaps, the lower the flow resistance encountered by the flushing liquid; conversely, the smaller the bearing gaps, the greater the flow resistance encountered by the flushing liquid. The flow resistance due to the bearing gaps also constitutes at least a part of the ambient pressure.

In an embodiment, in the process of flowing forward in the catheter 201 and flowing forward in the driving shaft 202, the first part of the flushing liquid also encounters flow resistance, and the flow resistance also constitutes at least a part of the ambient pressure.

In order to avoid causing discomfort to the human body, the amount of flushing liquid entering the human body via the catheter 201 should not be too large, and should be less than the amount flowing out via the flushing liquid outlet 246. On this basis, the pressure of the flushing liquid in the cooling circulation loop is maintained by the pressure maintenance module 600 to be greater than the ambient pressure of the pump head 205 in an operating state, which is why the flushing liquid is able to enter the catheter 201 having great resistance. Thus, the flow rate distribution of the flushing liquid according to the description hereinabove is able to be achieved by controlling the pressure of the flushing liquid in the cooling circulation loop by means of the pressure maintenance module 600.

In different clinical situations, the amount of flushing liquid required to enter the human body via the catheter 201 is different; however, in various scenarios, the amount of flushing liquid entering the human body via the catheter 201 is generally much less than the amount of flushing liquid flowing out via the flushing liquid outlet 246. Thus, the pressure of the flushing liquid in the cooling circulation circuit is controlled by the pressure maintenance module 600 to be slightly greater than the ambient pressure of the pump head 205 in operation.

As shown in Fig. 3 and Fig. 4, the rotor shaft 2047 is provided with a channel 2053 axially passing through the rotor shaft, the flushing chamber 2042 and the liquid isolating chamber 2050 are in communication with each other by means of the channel 2053 (the proximal end of the channel 2053 being located in the flushing chamber 2042, and the distal end being located in the liquid isolating chamber 2050), the catheter 202 and the flushing liquid inlet 2043 are in communication with the flushing chamber 2042, and the flushing liquid outlet 2046 is in communication with the liquid isolating chamber 2050. In this way, the flushing liquid entering the flushing chamber 2042 passes through the channel 2053 and flows backward to the liquid isolating chamber 2050, and finally flows out via the flushing liquid outlet 2046, such that the flushing liquid is able to continuously cools the rotor 2046 in a circulating manner.

A first bearing 235 for rotatably supporting the distal end of the rotor shaft 2047 is provided in the accommodating chamber 2049, the first bearing 235 provides partial liquid isolation between the flushing chamber 2042 and the liquid isolating chamber 2050, and the flushing liquid inlet 2043 and the flushing liquid outlet 2046 are respectively located on either side of the bearing 235. The liquid isolation effect of the first bearing 235 prevents the flushing liquid entering the flushing chamber 2043 from flowing directly to the flushing liquid outlet 2046, and restricts the flushing liquid to mainly flowing backward via the channel 2053, so as to achieve cooling of the rotor 2046.

The diameter of the rotor shaft 2047 is less than the inner diameter of the first bearing 235, a first gap 2351 is present between the rotor shaft and the first bearing, and the flushing liquid is able to pass through the first gap 2351 to achieve lubrication of the first bearing 235. The cross-sectional area of the first gap 2351 is less than the cross-sectional area of the channel 2053, so that the flushing liquid mainly flows backward via the channel 2053 having a larger cross-sectional area, so as to ensure the flow rate of the flushing liquid for circulating cooling.

A second bearing 236 configured for rotatably supporting the proximal end of the rotor shaft 2047 is also provided in the accommodating chamber 2049, and the second bearing 236 is located at the proximal end of the first bearing 235; and the second bearing and the first bearing 235 cooperate to rotatably support two ends of the rotor 2046, facilitating keeping the rotation stability of the rotor 2046. Likewise, the diameter of the rotor shaft 2047 is less than the inner diameter of the second bearing 236, a second gap 2362 is present between the rotor shaft 2047 and the second bearing 236, and the flushing liquid is able to pass through the second gap 2362 to achieve lubrication of the second bearing 236.

The second bearing 236 is provided with a flow passage hole 2361 axially passing through the second bearing, and the cross-sectional area of the flow passage hole 2361 is larger than the cross-sectional area of the second gap 2362. In this way, the area of the hole through which the flushing liquid reversely flows forward is able to be increased, reducing the resistance encountered by the flushing liquid, achieving a large circulation flow rate.

As shown in Fig. 4, the pressure maintenance module 600 includes the flushing liquid source 602 in communication with the cooling circulation loop by means of a replenishment line 601, and a pump 603 provided on the replenishment line 601 to replenish, into the cooling circulation loop, flushing liquid provided by the flushing liquid source 602.

In an embodiment, the flushing liquid entering the catheter 201 and consumed is replenished by an additionally provided liquid source, i.e. the flushing liquid source 602. The pressure of the flushing liquid in the cooling circulation loop is maintained by a further driving mechanism, i.e. the pump 603. In this way, the flushing liquid circulation and the flushing liquid pressure maintenance are implemented by the circulation driving member 503 and the pump 603, respectively, and the pressure required for the flushing liquid circulation and the pressure required for the flushing liquid pressure maintenance are different (in general, the pressure required for the flushing liquid circulation being less than the pressure required for the flushing liquid pressure maintenance). Thus, the flushing liquid circulation and the flushing liquid pressure maintenance are separately driven, which is able to simplify the control of the circulation driving member 503 and the control of the pump 603, and makes the control of the flushing liquid pressure and the control of the flushing liquid flow rate more accurate.

In order to facilitate line connection, the cooling circulation module 600 further includes a temporary storage container 504, and the flushing liquid inlet tube 501, the flushing liquid outlet tube 502, and the replenishment line 603 all communicate with the temporary storage container 504. The lines may communicate with the temporary storage container 504 using a Luer taper, providing convenience for line connection.

To further improve the flushing liquid pressure maintenance or control accuracy, the temporary storage container 504 is able to withstand at least the pressure compensation by the pressure maintenance module 600 without a change in volume. Further, the temporary storage container 504 is a solid tank having high pressure resistance and maintaining a constant volume under normal pressure. Alternatively, in some embodiments, a three-way connector may directly be used as the temporary storage container 504, three ports of the three-way connector are connected to the flushing liquid inlet tube 501, the flushing liquid outlet tube 502, and the replenishment line 603, respectively, and the internal chambers of the three-way connectors are configured to construct the described chamber maintaining a constant volume when withstanding pressure. Thus, configuring the temporary storage container 504 to be a three-way connector greatly simplifies a flushing line.

Practice proves that by providing in the cooling circulation loop the temporary storage container 504 having a constant volume, the accuracy of the flushing liquid pressure maintenance or control is greatly improved. This effect is surprising, since before this design is adopted, the flushing liquid inlet tube 501, the flushing liquid outlet tube 502, and the replenishment line 603 are connected by flexible connectors, and after multiple tests, the research personnel always found that it is difficult to make the control of the flushing liquid pressure stable or achieve an expected effect.

The inventors sought the reason why this design led to the described effects, but it is unclear at present. The possible principle was guessed as follows:

In order to facilitate the deployment and organization of clinical lines, the flushing liquid inlet tube 501 and the flushing liquid outlet tube 502 constructing the cooling circulation loop are generally flexible tubes, and the diameters of flexible tubes expand under the effect of a high flushing liquid pressure, causing a change in the volume of the cooling circulation loop. The volume of the cooling circulation loop changes, while the pressure maintenance module 600 still operates in an original state (for example, the rotational speed of the pump), which may cause the pressure of the flushing liquid in the cooling circulation loop to change (decrease), causing the amount of flushing liquid entering the catheter 201 to be unstable.

On the contrary, although the provision of the constant-volume temporary storage container 504 in the cooling circulation loop (the volume of the temporary storage container 504 forming a part of the cooling circulation loop) does not avoid the diameter expansion of the flushing liquid inlet tube 501 and the flushing liquid outlet tube 502, compared with the volumes of the flushing liquid inlet tube 501 and the flushing liquid outlet tube 502, the volume of the temporary storage container 504 is larger, and the volume of the temporary storage container 504 is not prone to change. In this way, the volume change rate of the cooling circulation loop is able to be greatly reduced. In the embodiment illustrated in Fig. 4, the pump 603, which serves to maintain the pressure of the cooling circulation loop, is connected to the temporary storage container 504 by means of the replenishment line 601. In this way, the pump pressure of the pump 603 directly acts on the temporary storage container 504, rather than acts on the flushing liquid inlet tube 501 and the flushing liquid outlet tube 502, which may also has the effect of reducing the diameter change of the flushing liquid inlet tube 501 and the flushing liquid outlet tube 502 to some extent.

In conclusion, by providing in the cooling circulation loop the temporary storage container 504 having a constant volume, the problem of a change in the volume of the cooling circulation loop is at least partially solved, improving the accuracy of flushing liquid pressure maintenance or control.

As shown in Fig. 4, in one embodiment, the circulation driving member 503 is a pump arranged on the flushing liquid inlet tube 501 and/or the flushing liquid outlet tube 502 and is different from the pump 603 (for distinguishing, this pump being defined as a first pump, and the pump 603 being defined as a second pump). Because technology of pump control is comparatively mature, using a pump as the circulation driving member 503 is able to simplify the control.

Research found that, high-speed rotation of the rotor 2046 in an environment submerged in flushing liquid generates bubbles. Gas originally blended in the flushing liquid precipitates due to agitation by the rotor 2046 and temperature rise so that bubbles are generated. Clinically it is undesirable to let the bubbles enter the human body, thus it is necessary to catch the generated bubbles.

In an embodiment, the cooling circulation loop is provided with a bubble filter 258 for filtering or catching bubbles to prevent bubbles from entering the human body via the catheter 201. Further, the bubble filter 258 is provided on the flushing liquid inlet tube 501. This design facilitates the arrangement of the bubble filter 258, avoiding interference with other structures.

In addition, bubbles are mainly generated in the liquid isolating chamber 2050, then the bubbles flow out along with the flushing liquid via the flushing liquid outlet 246, then circulate in the flushing liquid outlet tube 502 and the flushing liquid outlet tube 501, and enter the flushing chamber 2042 again via the flushing liquid inlet 2043. Hence, the bubbles do not enter the human body via the catheter 201 from being generated till entering the flushing chamber 2042 again via the flushing liquid inlet 2043. This means that entry of bubbles in the body is able to be substantially avoided before the bubbles enter the flushing chamber 2042. Thus, this purpose is able to be achieved by providing the bubble filter 258 on the flushing liquid inlet tube 501.

The bubble filter 258 may adopt any suitable existing structure, in an embodiment, a mesh filter or a membrane filter, which is not limited in the present invention. The mesh filter or membrane filter is housed in a housing structure; in order to reduce the resistance of the mesh filter or membrane filter to the flow of flushing liquid, the flow passage area of the mesh filter or membrane filter is able to be increased as much as possible; and the housing for housing the mesh filter or membrane filter is able to be a flat and expanded structure.

Practice dictates that using the flushing solution of the embodiment not only greatly reduces the high temperature of flushing liquid, but also cools the driving assembly 100. Specifically, as shown in Fig. 3, the motor shaft 1021 is provided with a rotary support 105, and the driving member 103 is arranged on the inner wall of the rotary support 105. A bearing chamber 109 is provided inside the motor housing 101, the bearing chamber 109 is connected to the motor 102 by means of a flange 111, and the bearing chamber 109 is internally provided with a bearing 110 for rotatably supporting the rotary support 105. Thus, rotating components, such as the motor 102, the driving member 103, and the bearing 110 inside the driving assembly 100 are parts having great heat generation. To solve the heat dissipation problem of these components having great heat generation, the motor 102 is in contact with the bearing chamber 109 by means of the flange 111. In this way, during operation, the driving member 103, the rotary support 105, the bearing 110, the bearing chamber 109, the flange 111, and the motor 102 are connected and in physical contact; by means of the described physical contact, a heat conducting path is formed from the motor 102, the flange, the bearing chamber 109, the bearing 110, and the rotary support 105 to the driving member 103, such that heat of main heat generation components, i.e. the motor 102, the bearing 110, and the driving member 103 of the driving assembly 110 is carried away by a high flow rate of flushing liquid circulating in the driving catheter handle 204, and is further dissipated into the external air when the flushing liquid is involved in extracorporeal circulation, achieving cooling of the described components. The driving member 103 is the component closest to the flushing liquid; thus, the heat of the driving member 103 is transferred to the flushing liquid in the liquid isolating chamber 2050 via the liquid isolating wall 2048, such that the driving member is cooled and forms a comparatively low temperature, such that heat of other components in physical contact is transferred to the driving member 103 and is then transferred to the liquid isolating chamber 2050 via the liquid isolating wall 2048, achieving cooling.

Since main heat generation components of the driving assembly 100 are cooled by the flushing liquid in the driving catheter handle 204, the heat dissipation design of the driving assembly 100 is able to be simplified. As shown in Fig. 3, the motor housing 101 is made of a plastic material. Compared with a housing made of metal, the plastic motor housing 101 has a lower cost, lighter weight, and better hand feeling, but has a poorer heat dissipation effect. However, the poorer heat dissipation effect is clinically better. The reason is: the outer surface of the motor housing 101 is inevitably in contact with the human body. When becoming in contact with a part of the human body, for example, the hand, the motor housing 101 made of a material with good thermal conductivity, such as metal, will fast conduct heat to the human body, and thus the part of the human body may be burned. On the contrary, with the same surface temperature, due to poor thermal conductivity, the plastic motor housing 101 would not damage the human body even if it is touched by the human body.

It should be understood that the description hereinabove is for purposes of illustration and not limitation. Many embodiments and many applications beyond the examples provided will be apparent to a person skilled in the art from a review of the description hereinabove. Thus, the scope of the present teachings should not be determined with reference to the description hereinabove, but should instead be determined with reference to the appended claims, along with the full scope of equivalents to which such claims are entitled.

For the purpose of comprehension, all articles and references, including publication of patent applications and announcement, are incorporated herein by reference. The omission of any aspect of the subject matter disclosed herein in the preceding claims is not intended to forego the subject matter, and it should not be deemed that the inventors do not consider the subject matter as not being a part of the disclosed invention subject matter.

## Claims

1. A catheter pump, comprising:
a motor;
a catheter;
a driving shaft, wherein the driving shaft rotatably passes through the catheter;
a pump head, wherein the pump head comprises a pump housing connected to a distal end of the catheter, and an impeller housed in the pump housing, wherein the impeller is connected to a distal end of the driving shaft so as to be driven to rotate to pump blood;
a driving catheter handle, wherein the driving catheter handle comprises: a coupling housing connected to a proximal end of the catheter and detachably connected to the motor, and a rotor which is able to be driven by the motor, wherein a proximal end of the driving shaft is connected to the rotor, and an accommodating chamber for rotatably supporting the rotor therein is formed in the coupling housing; wherein the catheter is in communication with the accommodating chamber, and the coupling housing is provided with a flushing liquid inlet and a flushing liquid outlet in communication with the accommodating chamber;
a cooling circulation module, wherein the cooling circulation module comprises: a flushing liquid inlet tube in communication with the flushing liquid inlet, and a flushing liquid outlet tube in communication with the flushing liquid outlet, wherein the flushing liquid inlet tube, the accommodating chamber, and the flushing liquid outlet tube form a cooling circulation loop, and a first pump for driving flushing liquid to flow is provided on the cooling circulation loop; and
a pressure maintenance module, wherein the pressure maintenance module comprises a flushing liquid source in communication with the cooling circulation loop by means of a replenishment line, and a second pump provided on the replenishment line to replenish flushing liquid provided by the flushing liquid source into the cooling circulation loop.

2. The catheter pump as claimed in claim 1, wherein the second pump is configured for maintaining a pressure of flushing liquid in the cooling circulation loop to be greater than an ambient pressure of the pump head during operation.

3. The catheter pump as claimed in claim 1 or 2, wherein flushing liquid entering the accommodating chamber via the flushing liquid inlet has one part entering the catheter and is entirely discharged via the pump head to a human body, and the other part passing through the rotor and then being discharged via the flushing liquid outlet.

4. The catheter pump as claimed in claim 3, wherein an amount of flushing liquid entering the catheter is less than an amount of flushing liquid discharged from the flushing liquid outlet.

5. The catheter pump as claimed in claim 1, wherein the cooling circulation module further comprises a temporary storage container, the flushing liquid inlet tube, the flushing liquid outlet tube, and the replenishment line are all in communication with the temporary storage container, and the temporary storage container is able to withstand at least pressure compensation by the pressure maintenance module without a change in volume.

6. The catheter pump as claimed in claim 5, wherein the temporary storage container comprises a solid tank or a three-way connector.

7. The catheter pump as claimed in claim 1, wherein the cooling circulation loop is provided with a bubble filter.

8. The catheter pump as claimed in claim 7, wherein the bubble filter is provided on the flushing liquid inlet tube.

9. The catheter pump as claimed in claim 1, wherein the rotor comprises a rotor shaft connected to the proximal end of the driving shaft, and the rotor shaft has a channel axially passing through the rotor shaft; wherein the driving catheter handle further comprises a flushing support and a liquid isolating wall which are arranged in the coupling housing, the flushing support defines a flushing chamber, and the liquid isolating wall defines a liquid isolating chamber; wherein the flushing chamber is in communication with the liquid isolating chamber by means of the channel; wherein the catheter and the flushing liquid inlet are in communication with the flushing chamber, and the flushing liquid outlet is in communication with the liquid isolating chamber.

10. The catheter pump as claimed in claim 9, wherein the motor drives a driving member to transfer rotational power of the motor to a driven member coupled to the driving member, the liquid isolating wall is located between the driving member and the driven member, and heat of the driving member is transferred to flushing liquid in the liquid isolating chamber via the liquid isolating wall.

11. The catheter pump as claimed in claim 10, wherein a motor shaft of the motor is connected to a rotary support, and the driving member is arranged on the inner wall of the rotary support; wherein the motor is housed in a motor housing, the motor housing is internally provided with a bearing chamber, the bearing chamber is connected to the motor by means of a flange, and the bearing chamber is internally provided with a bearing for rotatably supporting the rotary support.

12. The catheter pump as claimed in claim 10 or 11, wherein the material of the motor housing is plastic.

13. The catheter pump as claimed in claim 9, wherein the accommodating chamber is internally provided with a first bearing for rotatably supporting a distal end of the rotor shaft, the first bearing provides partial liquid isolation between the flushing chamber and the liquid isolating chamber, and the flushing liquid inlet and the flushing liquid outlet are respectively located on either side of the first bearing.

14. The catheter pump as claimed in claim 13, wherein a first gap is present between the first bearing and the rotor shaft.

15. The catheter pump as claimed in claim 14, wherein a cross-sectional area of the first gap is less than a cross-sectional area of the channel.

16. The catheter pump as claimed in claim 13, wherein the accommodating chamber is internally provided with a second bearing for rotatably supporting a proximal end of the rotor shaft, and a second gap is present between the second bearing and the rotor shaft.

17. The catheter pump as claimed in claim 16, wherein the second bearing is provided with a flow passage hole axially passing through the second bearing, a cross-sectional area of the flow passage hole being larger than a cross-sectional area of the second gap.
